# EUROPÄISCHE PATENTSCHRIFT

(11) **EP 2 445 557 B1**
(45) Veröffentlichungstag und Bekanntmachung des Hinweises auf die Patenterteilung: **29.05.2013**
(21) Anmeldenummer: 10742401.2
(22) Anmeldetag: 23.06.2010
(51) Int. Cl.: A61M 11/00, A61M 15/00, B01D 45/16

(54) **TURBOINHALATOR**
TURBO-INHALER
TURBO INHALATEUR

(30) Priorität: 24.06.2009 DE 102009030185
(43) Veröffentlichungstag der Anmeldung: 02.05.2012
(73) Patentinhaber: United Therapeutics Corporation, Silver Spring, Maryland 20910 (US)
(72) Erfinder: Kern, Joachim, 63820 Elsenfeld (DE)
(74) Vertreter: HOFFMANN EITLE
(86) Internationale Anmeldenummer: PCT/DE2010/000715
(87) Internationale Veröffentlichungsnummer: WO 2010/149144

(56) Entgegenhaltungen:
- EP-A1- 0 504 459
- CA-A1- 2 207 219
- DE-A1-102006 026 786
- GB-A- 2 224 446
- US-A- 3 885 934
- US-A- 5 881 715

## Beschreibung

Die Erfindung bezieht sich auf einen Turboinhalator, bestehend aus einem Wirkflüssigkeitsbehälter, in dem sich eine Flüssigkeit mit einem darin gelösten Wirkstoff befindet sowie einem Vernebler, durch den Flüssigkeit in ein Aerosol umwandelbar ist und in ein Schaufelgehäuse einbringbar ist, in dem eine Begrenzungskalotte aufgehängt ist, deren konkave Seite zum Vernebler hin gerichtet ist und einem Abluftrohr, das im Bereich der konvexen Seite der Begrenzungskalotte an das Schaufelgehäuse angeschlossen ist und einer Zuluftführung, durch die Zuluft zwischen Schaufelgehäuse und dem Wirkstoffbehälter einführbar ist.

Auf aktuellem Stand der Technik sind Inhalatoren eine mittlerweile gut bewährte Methode, um ein Arzneimittel und andere Wirkstoffe in flüssiger Form zusammen mit der Atemluft in die Lunge von Menschen und Tieren zu transportieren, von wo sie sehr schnell und verlustarm in die Blutbahn übertragen wird.

Die bislang üblichen, mechanischen Zerstäuber, die die Flüssigkeit durch eine Düse pressen, um sie in ein Aerosol umzuformen, also einen Nebel zu bilden, sind derzeit sehr oft durch einen zumeist kegelförmigen Wirkstoffbehälter ersetzt, unterhalb dessen ein Piezokristall als Vernebler angeordnet ist, der eine Ultraschallschwingung erzeugt und sie auf die Wirkflüssigkeit überträgt. Dadurch bildet sich auf dem Flüssigkeitsspiegel ein Sprudel, der die Wirkflüssigkeit als Aerosol absondert, also ein Gemisch als Luft und feinsten Tröpfchen der Wirkflüssigkeit. Dabei ist es das Ziel, möglichst kleine Tropfen zu erzeugen, deren Durchmesser vier Mikrometer nicht überschreiten sollte und bei dem ein möglichst großer Anteil der Tröpfchen einen Durchmesser nahe zwei Mikrometer hat.

Auf aktuellem Stand der Technik zeigt die DE 19838711 eine Begrenzungskalotte oberhalb des Sprudels, die ihre konkave Seite dem Sprudel zuwendet. Diese Begrenzungskalotte fängt Spritzer aus dem Wirkstoffbehälter auf, die sich insbesondere bei sinkendem Flüssigkeitsspiegel bilden, und leitet diese Flüssigkeitsmengen in den Wirkstoffbehälter zurück.

In die kalottenförmige Vertiefung der Begrenzungskalotte werden auch die aus dem Sprudel austretenden Aerosole hineingetrieben, gleiten an der Innenfläche entlang und wechseln beim Verlassen der Kalotte sehr schnell ihre Richtung, vollziehen also etwa einen 180 Grad Schwenk und durchströmen dann den Spalt zwischen der Begrenzungskalotte und dem Gehäuse, in dem diese befestigt ist. Durch diesen schnellen Richtungswechsel werden die größten Tröpfchen des Aerosols an die Wand des Gehäuses geschleudert, wo sie haften bleiben und zu Tröpfchen agglomieren, die wieder in den Wirkstoffbehälter zurückfallen. Die kleinen Tröpfchen des Aerosols werden hingegen von der Atemluft mitgerissen und aus dem Abluftrohr oberhalb der Begrenzungskalotte von der Atemluft herausgesagt.

Als eine weitere Verfeinerung dieses Prinzips präsentiert die DE 101 01 454 zusätzliche Prallplatten, die im Zwischenraum zwischen der Begrenzungskalotte und dem Gehäuse quer zum Strom angeordnet sind und das Aerosol in den Spalt zwischen Prallplatte und Begrenzungskalotte zwängen. Diese zusätzlichen Prallflächen sorgen für eine weitere Reduzierung des Anteils von Tröpfchen mit relativ großem Durchmesser, wobei ein Durchmesser von 5 Mikrometer und mehr als groß eingestuft wird.

Ein Dosierinhalator aufweisend alle Merkmale des Oberbegriffs des Anspruchs 1 ist auch aus dem Dokument DE 10 2006 026786 bekannt.

Ein Nachteil der geschilderten Anordnungen ist jedoch, dass in den Verlauf der an den Austritt des Gehäuses anschließenden Rohre immer noch so zahlreiche, große Aerosoltröpfchen mitgerissen werden, dass sich in Krümmungen des Rohres oder an Verbindungsstellen von zwei Rohren feine Tröpfchen niederschlagen, sich mit weiteren Tropfen verbinden und schließlich mit bloßem Auge sichtbare Flüssigkeitstropfen bilden. Insbesondere, wenn das Rohr durchsichtig ist, wird auf diese Weise sichtbar, dass nicht der gesamte, evtl. sehr teure - Wirkstoff in die Lunge transportiert wird, sondern ein Teil verloren geht.

Ein weiterer Nachteil ist, dass diese Tröpfchen in der Rohrstrecke vor einer weiteren Verwendung des Inhalators sorgfältig beseitigt werden müssen, um eine Vermischung mit dem folgenden Wirkstoff einer weiteren Anwendung zu vermeiden.

Der wesentliche Nachteil dieses Prinzips ist der durch die Verengungen bei den Prallplatten bedingte, recht hohe Strömungswiderstand für die Atemluft, wodurch die Nutzer beim Atmen behindert werden. Da die Nutzer oft durch eine Erkrankung geschwächt sind, ist das nicht nur für sie unkomfortabel sondern kann sogar zu einer Änderung des Atemverhaltens wie z.B. flachem und unregelmäßigem Atmen führen, was wiederum die Wirksamkeit der Inhalation beeinträchtigt.

Auf diesem Hintergrund hat sich die Erfindung die Aufgabe gestellt, einen Inhalator zu entwickeln, der allzu große Aerosoltröpfchen aus dem Aerosol aussondert und in den Wirkstoffbehälter zurück leitet und dabei den Strömungswiderstand für das Atmen nur sehr wenig erhöht.

Als Lösung dieser Aufgabe lehrt die Erfindung, dass wenigstens eine Leitschaufel auf der konkaven Seite der Begrenzungskalotte aufgesetzt ist und darauf spiralförmig verläuft.

Das entscheidende Element der Erfindung sind also die Leitschaufeln, die der Strömung einen Drall verleihen, sie also zusätzlich in eine Rotation um ihre Längsachse versetzen. Dadurch wird auf die Aerosoltröpfchen eine Fliehkraft ausgeübt, die umso größer ist je größer der Durchmesser und damit die Masse des Aerosoltröpfchens ist. Durch diese Fliehkraft werden vor allem die relativ großen Aerosoltröpfchen an den Rand des Schaufelgehäuses gedrückt, von wo aus sie direkt in den Wirkstoffbehälter zurückfließen können.

Im Gegensatz zum bekannten Stand der Technik sind die Leitschaufeln weder quer zum Luftstrom ausgerichtet, wie z. B. die Impaktorwände der DE 10101454 , noch sind sie in Richtung des Luftstromes orientiert, wie das vom Träger der Begrenzungskalotte bekannt ist. Vielmehr sind die zusätzlichen Leitschaufeln schräg zum Luftstrom ausgerichtet, sodass er seine Richtung ändern muss. Zusätzlich verlaufen die Leitschaufeln spiralförmig auf der konkaven Seite der Begrenzungskalotte, wodurch die Richtung des Luftstromes und des darin enthaltenen Aerosols nicht nur ein einziges Mal beim Auftreffen auf die Leitfläche geändert wird, sondern laufend, da sich auch die effektive Neigung der Prallfläche durch ihre Spiralform laufend ändert.

Durch diese kontinuierlichen Richtungsänderung wird dem Luftstrom ein Drall verliehen. Er rotiert also um seine Längsachse, so dass insbesondere auf die relativ großen Aerosoltröpfchen Fliehkräfte wirken, die sie aus dem Luftstrom heraus auf die Innenfläche des Schaufelgehäuses drücken, so dass sie dort haften bleiben und zu Tröpfchen agglomieren, die an der Gehäusewand entlang in den Wirkstoffbehälter zurück laufen und später als Aerosol wieder zurück kommen.

Dieser Effekt zur Abscheidung von relativ großen Aerosoltröpfchen lässt sich auch als komprimierter Wirbel erklären, der sich oberhalb der Kalotte mit den aufgesetzten Leitschaufeln bildet.

Dieser Wirbel ist homogen und deshalb besonders effizient, wenn zahlreiche Leitschaufeln über den Umfang hinweg etwa gleichmäßig verteilt sind. Sie wirken dann etwa so ähnlich wie bei einer Turbine, dem Propeller eines Schiffes oder eines Flugzeuges, jedoch mit dem wesentlichen Unterschied, dass hier der Propeller ortsfest ist und von dem Aerosol angeströmt wird. Nach dem Verlassen der Leitschaufeln weist der Aerosolstrom einen Drall auf. Er rotiert also um die Längsachse seiner Strömungsrichtung.

Wenn dieser Strom beim Eintreten in das Abluftrohr von dem relativ großen Durchmesser des Schaufelgehäuses auf den relativ kleinen Durchmesser des Abluftrohres reduziert wird, bleibt die kinetische Energie dieser Prallbewegung im wesentlichen erhalten. Durch die deutliche Reduzierung des Durchmessers nimmt die Geschwindigkeitskomponente der Aerosoltröpfchen tangential zur Hauptströmungsrichtung deutlich zu, wodurch sich die größten Tröpfchen am Gehäuse niederschlagen, dort zu größeren Tropfen agglomieren, die durch die Leitschaufeln hindurch wieder in den Wirkflüssigkeitsbehälter zurückfallen und dort erneut in Aerosol umgewandelt werden.

Dieser Effekt gilt selbstverständlich auch für nur eine einzige Leitschaufel. Eine solche einzige Leitschaufel kann sich wie eine archimedische Schraube um die Begrenzungskalotte herumziehen. Diese Variante hat jedoch als Einschränkung, das der "Rückweg" von niedergeschlagenen Aerosoltröpfchen sehr lang ist, weshalb eine größere Anzahl von Leitschlaufen bevorzugt wird. Mit einer wachsenden Anzahl von Leitschaufeln steigert sich auch deren vorteilhafte Wirkung auf die größten Aerosoltröpfchen. Ein Maximum dieser Wirkung ist dann erreicht, wenn die gesamte Querschnittsfläche des Schaufelgehäuses in Strömungsrichtung mit Leitschaufeln abgedeckt ist.

Vier bis sechs Leitschaufeln haben sich in der Praxis als ein sehr guter Wert erwiesen.

Eine weitere Erhöhung der Anzahl der Leitschaufeln erhöht nur den Strömungswiderstand ohne die abscheidende Wirkung weiter zu verstärken. Dabei ist eine mittlere Steigung der spiralförmig auf der Begrenzungskalotte verlaufenden Leitschaufeln in einer Größenordnung von etwa 45 Grad die bevorzugte Ausführungsvariante. Größere oder kleinere Mittelwerte sind ebenfalls möglich, werden jedoch in der Regel nicht so gut beim Aufbau des zum Abscheiden der größten Aerosoltröpfchen erforderlichen Dralls im Luftstrom helfen. Dabei ist zu beachten, dass die Leitschaufel stets spiralförmig um die Kalotte herum verläuft und deshalb von nur einem Betrachtungspunkt aus gesehen eine wechselnde Steigung aufweist.

Dabei ist zu bevorzugen, dass die Leitschaufeln gleichmäßig über den Umfang der Begrenzungskalotte verteilt sind, weil dann auch die Einwirkung auf den Luftstrom gleichmäßig ist.

Der Luftstrom durch den erfindungsgemäßen Turboinhalator verläuft in seinem Inneren entlang einer Längsachse die sich vom Vernebler zum Abluftrohr erstreckt. In Bezug auf diese Längsachse können die Querschnitte der Elemente des Turboinhalators im allgemeinsten Fall beliebig sein. Zu bevorzugen ist jedoch ein auf die Längsachse bezogen rotationssymmetrischer Aufbau. Das Schaufelgehäuse, die Begrenzungskalotte und der Wirkstoffbehälter weisen dann kreisförmige Querschnitte auf und sind zueinander koaxial angeordnet.

Bei einem erfindungsgemäßen Turboinhalator kann die Begrenzungskalotte durch einen Träger mit dem Schaufelgehäuse verbunden werden. Es ist jedoch zu bevorzugen, dass wenigstens eine Leitschaufel so lang ausgelegt ist, dass sie sich zumindest mit ihrer Spitze bis zum Schaufelgehäuse erstreckt und deshalb neben ihrer Funktion als Leitfläche für den Luftstrom auch die mechanische Befestigung der Begrenzungskalotte übernimmt.

Wie erwähnt ist es eine bevorzugte Ausführungsform, dass - quer zur Strömungsrichtung gesehen - der Raum zwischen dem Schaufelgehäuse und der Begrenzungskalotte vollständig mit Leitschaufeln ausgefüllt ist. Dann hat - in Richtung der Längsachse gesehen - die freie Kante jeder Leitschaufel, die zum Abluftrohr hinweist, einen Abstand zu der freien Kante der benachbarten Leitschaufel, die zum Vernebler hinweist. Es ist jedoch alternativ auch möglich, dass sich die Leitschaufeln in Richtung der Längsachse etwas überlappen, sodass jede Leitschaufel mit ihrer freien, zum Abluftrohr weisenden Kante etwas über die freie Kante der benachbarten Leitschaufel, die zum Vernebler hinweist, hinweg ragt.

In einer weiteren Variante können die Leitschaufeln in ihrer Steigung verändert werden, indem sie um eine quer zur Längsachse ausgerichtete Schwenkachse verschwenkbar sind. Durch eine Vergrößerung des spitzen Winkels zwischen den Leitschaufeln und der Längsachse vergrößert sich auch die Rotationsgeschwindigkeit des Luftstroms. In der Praxis wird diese Variante eher selten sein.

Im Folgenden sollen weitere Einzelheiten und Merkmale der Erfindung anhand eines Beispiels näher erläutert werden. Dieses soll die Erfindung jedoch nicht einschränken, sondern nur erläutern. Es zeigt in schematischer Darstellung:

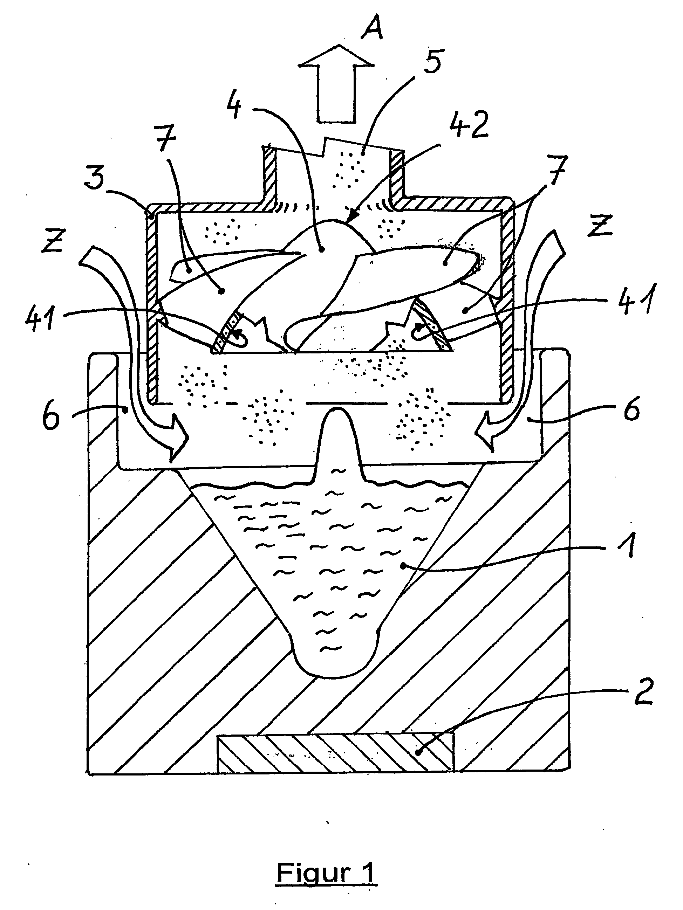

Figur 1 Schnitt durch einen Turboinhalator

In **Figur 1** ist ein erfindungsgemäßer Turboinhalator entlang der Längsachse 5 - 2 vom Abluftrohr 5 bis zum Vernebler 2 geschnitten dargestellt. An seiner untersten Seite ist der Vernebler 2 angeordnet, hier ein Piezokristall, der Ultraschall erzeugt, welches auf die Wirkflüssigkeit im darüber angeordneten Wirkflüssigkeitsbehälter 1 übertragen wird. Der Übersichtlichkeit halber sind in dieser Schnittzeichnung alle Steuerungselemente und sonstigen Funktionen zum korrekten Ansteuern des Verneblers 2 nicht dargestellt. Im Wirkflüssigkeitsbehälter 1 bildet sich in der Wirkflüssigkeit durch die Erregung mit Ultraschall ein Sprudel aus, von dem sich feine Tröpfchen als Aerosol absondern.

Zwischen dem Wirkflüssigkeitsbehälter 1 und dem darüber angeordneten Schaufelgehäuse 3 sind umlaufende Schlitze angeordnet, durch welche Zuluft Z eintritt und sich mit dem Aerosol vermischt.

Dieses Aerosol tritt in die Begrenzungskalotte 4 ein, die an ihren beiden Ecken aufgeschnitten dargestellt ist, um ihre konkave Seite 41 zu zeigen. Auf diese konkave Seite werden die größeren Partikel des Aerosols geschleudert, dort seitlich abgelenkt und zu dem Schlitz zwischen der Begrenzungskalotte 4 und dem Schaufelgehäuse 3 geführt. In Figur 1 ist gut nachvollziehbar, dass sich die an der konkaven Seite 41 der Begrenzungskalotte 4 gesammelten, größeren Aerosoltröpfchen zu Tropfen agglomieren und auch Spritzer aus dem Sprudel reflektiert werden, die wieder in den Wirkflüssigkeitsbehälter 1 herunter fallen.

Die übrig bleibenden, feineren Aerosoltröpfchen werden von der Abluft A, die beim Atmen durch das Abluftrohr 5 abgesaugt wird, in den Raum zwischen der konvexen Seite 42 der Begrenzungskalotte 4 und der Innenseite des Schaufelgehäuses 3 eingesaugt. Dort geraten sie in den Drall des Luftstromes, den auf die Leitschaufeln 7 erzeugen, was das entscheidende Merkmal der Erfindung ist. In Figur 1 ist gut zu sehen, wie sich die Leitschaufeln 7 spiralförmig auf der konvexen Seite 42 der Begrenzungskalotte 41 ausdehnen und dadurch den Luftstrom einen Drall verleihen.

Ebenfalls in Figur 1 ist gut nachvollziehbar, wie die Aerosole, die die turbinenartig angeordneten Leitschaufeln 7 durchlaufen, dem Drall des Luftstromes ausgesetzt sind, dessen Drehrichtung senkrecht zu der Längsachse 5 - 2 ausgerichtet ist und wie dadurch eine Fliehkraft auf sie ausgeübt wird.

Wenn die in Rotation um die Strömungshauptrichtung versetzten Aerosole von dem großen Durchmesser der Innenseite des Schaufelgehäuses 3 auf den sehr viel kleineren Durchmesser des Abluftrohres 5 reduziert werden, steigt ihre Umfangsgeschwindigkeit deutlich an, da die kinetische Energie im wesentlichen erhalten bleibt. Dadurch erhöht sich die wirksame Fliehkraft und es werden an der Eingangsstelle des Abluftrohres 5 weitere, größere Aerosolpartikel an der Wandung des Abluftrohres 5 angelagert. Sie sammeln sich dort mit anderen Aerosolpartikeln und bilden größere Tropfen, die dann wieder in den Wirkflüssigkeitsbehälter 1 zurückfallen.

### Bezugszeichenliste

| | |
|---|---|
| 1 | Wirkflüssigkeitsbehälter |
| 2 | Vernebler 2, unterhalb des Wirkflüssigkeitsbehälters 1, wandelt Flüssigkeit in ein Aerosol um |
| 3 | Schaufelgehäuse, oberhalb des Wirkflüssigkeitsbehälters 1, nimmt Aerosol auf |
| 4 | Begrenzungskalotte, oberhalb des Verneblers 2 |
| 41 | konkave Seite der Begrenzungskalotte 4, zum Vernebler 2 hin gerichtet, |
| 42 | konvexe Seite der Begrenzungskalotte 4 |
| 5 | Abluftrohr, gegenüber der konvexen Seite 42 der Begrenzungskalotte 4 angeordnet |
| 5-2 | Längsachse vom Abluftrohr 5 zum Vernebler 2 |
| 6 | Zuluftführung, führt Zuluft Z zwischen Schaufelgehäuse 3 und dem Wirkstoffbehälter 1 ein |
| 7 | Leitschaufel, auf der konvexen Seite 42 der Begrenzungskalotte 4 aufgesetzt |
| A | Abluft, aerosolhaltig, tritt durch Abluftrohr 5 aus |
| Z | Zuluft, tritt durch die Zuluftführung 6 in den Raum zwischen der Begrenzungskalotte 4 und dem Wirkflüssigkeitsbehälter 1 ein. |

## Patentansprüche

1. Turboinhalator, bestehend aus
- einem Wirkflüssigkeitsbehälter (1), in dem sich eine Flüssigkeit mit einem darin gelösten Wirkstoff befindet sowie
- einem Vernebler (2), durch den Flüssigkeit in ein Aerosol umwandelbar ist und
- in ein Schaufelgehäuse (3) einbringbar ist, in dem eine Begrenzungskalotte (4)aufgehängt ist, deren konkave Seite (41) zum Vernebler (2) hin gerichtet ist und
- einem Abluftrohn (5), das im Bereich der konvexen Seite (42) der Begrenzungskalotte (4) an das Schaufelgehäuse (3) angeschlossen ist und
- einer Zuluftführung (6), durch die Zuluft (Z) zwischen Schaufelgehäuse (3) und dem Wirkstoffbehälter (1) einführbar ist,
**dadurch gekennzeichnet, dass**
wenigstens eine Leitschaufel (7) auf der konvexen Seite (42) der Begrenzungskalotte (4) aufgesetzt ist und darauf spiralförmig verläuft.

2. Turboinhalator nach dem vorhergehenden Anspruch 1, **dadurch gekennzeichnet, dass** in Bezug auf eine Längsachse (5-2) vom Abluftrohr (5) bis zum Vernebler (2) das Innere des Schaufelgehäuses (3), die Begrenzungskalotte (4) und der Wirkstoffbehälter (1) rotationssymmetrisch geformt und koaxial zur Längsachse (5-2) angeordnet sind.

3. Turboinhalator nach Anspruch 2, **dadurch gekennzeichnet, dass** mehrere Leitschaufeln (7) gleichmäßig über den Umfang der Begrenzungskalotte (4) verteilt sind.

4. Turboinhalator nach Anspruch 3, **dadurch gekennzeichnet, dass** wenigstens eine Leitschaufel (7) mit dem Schaufelgehäuse (3) verbunden ist und als Träger für die Begrenzungskalotte (4) dient.

5. Turboinhalator nach einem der vorhergehenden Ansprüche, **dadurch gekennzeichnet, dass** die freie Kante jeder Leitschaufel (7), die zum Abluftrohr (5) hin weist, in Richtung der Längsachse (5-2) einen Abstand zu der freien Kante der benachbarten Leitschaufel (7) aufweist, die zum Vernebler (2) hin weist.

6. Turboinhalator nach einem der vorhergehenden Ansprüche, **dadurch gekennzeichnet, dass** jede Leitschaufel (7) mit ihrer freien Kante, die zum Abluftrohr (5) hin weist, in Richtung der Längsachse (5-2) über die freie Kante der benachbarten Leitschaufe (7), die zum Vernebler (2) hin weist, herüberragt.

7. Turboinhalator nach einem der vorhergehenden Ansprüche, **dadurch gekennzeichnet, dass** der Querschnitt des Schaufelgehäuses (3) quer zur Längsachse (5-2) vollständig mit Leitschaufeln (7) abgedeckt ist, wobei die einzelnen Leitschaufeln (7) zueinander in Richtung der Längsachse (5-2) beabstandet sind.

8. Turboinhalator nach einem der vorhergehenden Ansprüche, d**adurch gekennzeichnet**, dass zur Erhöhung der Rotationsgeschwindigkeit des Luftstromes der spitze Winkel zwischen den Leitschaufeln (7) und der Längsachse (5-2) vergrößerbar ist.

## Claims

1. Turbo-inhaler consisting of
- an active liquid container (1), in which a liquid having an active ingredient dissolved therein is located and
- a nebuliser (2), by means of which liquid can be converted into an aerosol and
- can be introduced into a vane housing (3), in which a boundary dome (4) is suspended, the concave side (41) of which is directed towards the nebuliser (2) and
- a waste air tube (5) which is connected to the vane housing (3) in the region of the convex side (42) of the boundary dome (4) and
- a supply air guide (6), through which supply air (Z) can be introduced between vane housing (3) and the active ingredient container (1),
**characterised in that**
at least one guide vane (7) is mounted on the convex side (42) of the boundary dome (4) and runs spirally thereon.

2. Turbo-inhaler according to the preceding claim 1, **characterised in that** with respect to a longitudinal axis (5-2) from the waste air tube (5) to the nebuliser (2), the interior of the vane housing (3), the boundary dome (4) and the active ingredient container (1) are formed to be rotationally symmetrical and arranged coaxially to the longitudinal axis (5-2).

3. Turbo-inhaler according to claim 2, **characterised in that** several guide vanes (7) are distributed uniformly over the periphery of the boundary dome (4).

4. Turbo-inhaler according to claim 3, **characterised in that** at least one guide vane (7) is connected to the vane housing (3) and serves as a support for the boundary dome (4).

5. Turbo-inhaler according to one of the preceding claims, **characterised in that** the free edge of each guide vane (7) which points towards the waste air tube (5) in the direction of the longitudinal axis (5-2) has a distance from the free edge of the adjacent guide vane (7) which points towards the nebuliser (2).

6. Turbo-inhaler according to one of the preceding claims, **characterised in that** each guide vane (7) with its free edge which points towards the waste air tube (5) in the direction of the longitudinal axis (5-2), projects beyond the free edge of the adjacent guide vane (7) which points towards the nebuliser (2).

7. Turbo-inhaler according to one of the preceding claims, **characterised in that** the cross-section of the vane housing (3) is covered completely with guide vanes (7) transversely to the longitudinal axis (5-2), wherein the individual guide vanes (7) are spaced from one another in the direction of the longitudinal axis (5-2).

8. Turbo-inhaler according to one of the preceding claims, **characterised in that** the acute angle between the guide vanes (7) and the longitudinal axis (5-2) can be increased to increase the rate of rotation of the air stream.

## Revendications

1. Turbo-inhalateur, constitué
- d'un récipient à liquide actif (1), où se trouve un liquide avec une substance active qui y est dissoute, et
- d'un nébuliseur (2), au moyen duquel le liquide peut être transformé en un aérosol, et
- être introduit dans un boîtier à pales (3), où est suspendu un dôme de retenue (4) dont la face concave (41) est opposée au nébuliseur (2), et
- d'un tuyau d'évacuation d'air (5), lequel est relié au boîtier à pales (3) au niveau de la face convexe (42) du dôme de retenue (4), et
- d'une amenée d'air (6), au moyen de laquelle de l'air entrant (Z) peut être introduit entre le boîtier à pales (3) et le récipient à principe actif (1),
**caractérisé en ce que**
au moins une pale directrice (7) est montée sur la face convexe (42) du dôme de retenue (4) où elle s'étend avec une forme hélicoïdale.

2. Turbo-inhalateur selon la revendication 1, **caractérisé en ce que** l'intérieur du boîtier à pales (3), le dôme de retenue (4) et le récipient à principe actif (1) présentent une forme à symétrie de rotation par rapport à un axe longitudinal (5-2) s'étendant du tuyau d'évacuation d'air (5) au nébuliseur (2), et sont disposés coaxialement audit axe longitudinal (5-2).

3. Turbo-inhalateur selon la revendication 2, **caractérisé en ce que** plusieurs pales directrices (7) sont régulièrement distribuées à la périphérie du dôme de retenue (4).

4. Turbo-inhalateur selon la revendication 3, **caractérisé en ce qu'**au moins une pale directrice (7) est reliée au boîtier à pales (3) et sert de support pour le dôme de retenue (4).

5. Turbo-inhalateur selon l'une des revendications précédentes, **caractérisé en ce que**, dans la direction de l'axe longitudinal (5-2), le bord libre de chaque pale directrice (7) opposé au tuyau d'évacuation d'air (5), est espacé du bord libre de la pale directrice (7) contiguë opposé au nébuliseur (2).

6. Turbo-inhalateur selon l'une des revendications précédentes, **caractérisé en ce que**, dans la direction de l'axe longitudinal (5-2), chaque pale directrice (7) dépasse par son bord libre opposé au tuyau d'évacuation d'air (5) du bord libre de la pale directrice (7) contiguë opposé au nébuliseur (2).

7. Turbo-inhalateur selon l'une des revendications précédentes, **caractérisé en ce que** la section transversale du boîtier à pales (3) est complètement couverte par les pales directrices (7) transversalement à l'axe longitudinal (5-2), les différentes pales directrices (7) étant espacées entre elles dans la direction de l'axe longitudinal (5-2).

8. Turbo-inhalateur selon l'une des revendications précédentes, **caractérisé en ce que**, pour élever la vitesse de rotation du flux d'air, l'angle aigu entre les pales directrices (7) et l'axe longitudinal (5-2) peut être agrandi.
